# EUROPEAN PATENT SPECIFICATION

(11) **EP 1 527 197 B1**
(45) Date of publication and mention of the grant of the patent: **18.06.2008**
(21) Application number: 03729923.7
(22) Date of filing: 07.04.2003
(51) Int. Cl.: C12Q 1/68

(54) **ASSOCIATION OF EDG5 POLYMORPHISM V286A WITH TYPE II DIABETES MELLITUS AND VENOUS THROMBOSIS/PULMONARY EMBOLISM AND THE USE THEREOF**
ZUSAMMENHANG VON EDG5 POLYMORPHISM V286A MIT DIABETES MELLITUS VON TYP II UND VENENTHROMBOSE/LUNGENEMBOLIE UND VERWENDUNG
ASSOCIATION DU POLYMORPHISME V286A DE L'EDG5 AVEC LES DIABETES SUCRES TYPE II ET LA THROMBOSE VEINEUSE/L'EMBOLIE PULMONAIRE ET UTILISATION ASSOCIEE

(30) Priority: 09.04.2002 EP 02007879
(43) Date of publication of application: 04.05.2005
(73) Proprietor: Sanofi-Aventis Deutschland GmbH, 65929 Frankfurt am Main (DE)
(72) Inventor: KOZIAN, Detlef, 65779 Kelkheim (DE); KOSTENIS, Evi, 36323 Grebenau (DE); SIEGLER, Karl-Ernst, 67069 Ludwigshafen (DE); JACOBS, Martina, 67574 Osthofen (DE); DELEUZE, Jean-Francois, F-77380 Combs La Ville (FR); RICARD, Sylvain, F-75013 PARIS (FR); MACE, Sandrine, F-78350 Jouy-en-Josas (FR)
(86) International application number: PCT/EP2003/003569
(87) International publication number: WO 2003/085130

(56) References cited:
- WO-A-99/54351
- DATABASE GENBANK [Online] Cds of the human lysosphingolipid receptor Edg 5, 1 January 1999 (1999-01-01) AN, S.: "Edg5, a human homolog of rat H218 that is a functional receptor for lysosphingolipids" Database accession no. AF034780 XP002227241 & MACLENNAN ET AL.: "Cloning and characterization of a putative G-protein coupled receptor potentially involved in development" MOLECULAR AND CELLULAR NEUROSCIENCES, SAN DIEGO, US, vol. 5, no. 5, June 1994 (1994-06), pages 210-209, XP002103008
- SESTI: "Insulin receptor substrate polymorphisms and type 2 diabetes mellitus" PHARMACOGENOMICS, vol. 1, no. 3, August 2000 (2000-08), pages 343-357, XP008012557
- BUSCH CP ET AL.: "Genetic determinants of type 2 diabetes mellitus" CLIN. GENET, vol. 60, 2001, pages 243-254, XP002227239
- RENDRIK ET AL.: "Genetic risk factors of venous thrombosis" HUMAN GENETIC, vol. 109, 2001, pages 369-384, XP002227240

### SEQUENZPROTOKOLL

<110> Aventis Pharma Deutschland GmbH
<120> Association of EDG5 polymorphism V286A with Type II Diabetes Mellitus and Venous Thrombosis/Pulmonary Embolism and the use thereof
<130> DEAV2002/0019
<140> 02007879.6
   <141> 2002-04-09
<160> 4
<170> PatentIn Ver. 2.1
<210> 1
   <211> 21
   <212> DNA
   <213> Artificial Sequence
<220>
   <223> Description of Artificial Sequence:oligonucleotide
<400> 1
   tccactgtcc tgcctctcta c 21
<210> 2
   <211> 20
   <212> DNA
   <213> Artificial Sequence
<220>
   <223> Description of Artificial Sequence:oligonucleotide
<400> 2
   tctccatgaa cccctctgcc 20
<210> 3
   <211> 353
   <212> PRT
   <213> Homo sapiens
<400> 3
   Met Gly Ser Leu Tyr Ser Glu Tyr Leu Asn Pro Asn Lys Val Gln Glu
   1 5 10 15
   His Tyr Asn Tyr Thr Lys Glu Thr Leu Glu Thr Gln Glu Thr Thr Ser
   20 25 30
   Arg Gln Val Ala Ser Ala Phe Ile Val Ile Leu Cys Cys Ala Ile Val
   35 40 45
   Val Glu Asn Leu Leu Val Leu Ile Ala Val Ala Arg Asn Ser Lys Phe
   50 55 60
   His Ser Ala Met Tyr Leu Phe Leu Gly Asn Leu Ala Ala Ser Asp Leu
   65 70 75 80
   Leu Ala Gly Val Ala Phe Val Ala Asn Thr Leu Leu Ser Gly Ser Val
   85 90 95
   Thr Leu Arg Leu Thr Pro Val Gln Trp Phe Ala Arg Glu Gly Ser Ala
   100 105 110
   Ser Ile Thr Leu Ser Ala Ser Val Phe Ser Leu Leu Ala Ile Ala Ile
   115 120 125
   Glu Arg His Val Ala Ile Ala Lys Val Lys Leu Tyr Gly Ser Asp Lys
   130 135 140
   Ser Cys Arg Met Leu Leu Leu Ile Gly Ala Ser Trp Leu Ile Ser Leu
   145 150 155 160
   Val Leu Gly Gly Leu Pro Ile Leu Gly Trp Asn Cys Leu Gly His Leu
   165 170 175
   Glu Ala Cys Ser Thr Val Leu Pro Leu Tyr Ala Lys His Tyr Val Leu
   180 185 190
   Cys Val Val Thr Ile Phe Ser Ile Ile Leu Leu Ala Ile Val Ala Leu
   195 200 205
   Tyr Val Arg Ile Tyr Cys Val Val Arg Ser Ser His Ala Asp Met Ala
   210 215 220
   Ala Pro Gln Thr Leu Ala Leu Leu Lys Thr Val Thr Ile Val Leu Gly
   225 230 235 240
   Val Phe Ile Val Cys Trp Leu Pro Ala Phe Ser Ile Leu Leu Leu Asp
   245 250 255
   Tyr Ala Cys Pro Val His Ser Cys Pro Ile Leu Tyr Lys Ala His Tyr
                   260                 265                 270
   Phe Phe Ala Val Ser Thr Leu Asn Ser Leu Leu Asn Pro Val Ile Tyr
               275                 280                 285
   Thr Trp Arg Ser Arg Asp Leu Arg Arg Glu Val Leu Arg Pro Leu Gln
           290                 295                 300
   Cys Trp Arg Pro Gly Val Gly Val Gln Gly Arg Arg Arg Val Gly Thr
   305                 310                 315                 320
   Pro Gly His His Leu Leu Pro Leu Arg Ser Ser Ser Ser Leu Glu Arg
                   325                 330                 335
   Gly Met His Met Pro Thr Ser Pro Thr Phe Leu Glu Gly Asn Thr Val
               340                 345                 350
   Val
<210> 4
   <211> 1062
   <212> DNA
   <213> Homo sapiens
<400> 4
   atgggcagct tgtactcgga gtacctgaac cccaacaagg tccaggaaca ctataattat 60
   accaaggaga cgctggaaac gcaggagacg acctcccgcc aggtggcctc ggccttcatc 120
   gtcatcctct gttgcgccat tgtggtggaa aaccttctgg tgctcattgc ggtggcccga 180
   aacagcaagt tccactcggc aatgtacctg tttctgggca acctggccgc ctccgatcta 240
   ctggcaggcg tggccttcgt agccaatacc ttgctctctg gctctgtcac gctgaggctg 300
   acgcctgtgc agtggtttgc ccgggagggc tctgcctcca tcacgctctc ggcctctgtc 360
   ttcagcctcc tggccatcgc cattgagcgc cacgtggcca ttgccaaggt caagctgtat 420
   ggcagcgaca agagctgccg catgcttctg ctcatcgggg cctcgtggct catctcgctg 480
   gtcctcggtg gcctgcccat ccttggctgg aactgcctgg gccacctcga ggcctgctcc 540
   actgtcctgc ctctctacgc caagcattat gtgctgtgcg tggtgaccat cttctccatc 600
   atcctgttgg ccatcgtggc cctgtacgtg cgcatctact gcgtggtccg ctcaagccac 660
   gctgacatgg ccgccccgca gacgctagcc ctgctcaaga cggtcaccat cgtgctaggc 720
   gtctttatcg tctgctggct gcccgccttc agcatcctcc ttctggacta tgcctgtccc 780
   gtccactcct gcccgatcct ctacaaagcc cactactttt tcgccgtctc caccctgaat 840
   tccctgctca accccgtcat ctacacgtgg cgcagccggg acctgcggcg ggaggtgctt 900
   cggccgctgc agtgctggcg gccgggggtg ggggtgcaag gacggaggcg ggtcgggacc 960
   ccgggccacc acctcctgcc actccgcagc tccagctccc tggagagggg catgcacatg 1020
   cccacgtcac ccacgtttct ggagggcaac acggtggtct ga                    1062

## Description

Endothelial differentiation gene (EDG) receptors are a new family of eight G protein-coupled receptors for the lysophospholipids lysophosphatitic acid and sphingosine-1-phosphate. The lysosphingolipid sphingosine 1-phosphate (S1P) regulates cell proliferation, apoptosis, motility, and neurite retraction (Pyne and Pyne, (2000) Biochem J 349: 385-402; MacLennan et al., (2001), J. of Neurosci. 14: 203-209). Its actions may be both, intracellular as a second messenger and extracellular as a receptor ligand. S1 P and the structurally related lysolipid mediator lysophosphatidic acid (LPA) signals through a set of G protein-coupled receptors known as EDG receptors. EDG5 (endothelial differentiation gene 5; also termed AGR16/H218) is a functional receptor for S1 P. The size of the EDG5 protein is 353 amino acids and the EDG5 gene is located on chromosome 19p13.2.

Mammalian EDG-5 Receptor homologs are described in WO 99/33972.

Developmental studies in Zebrafish have indicated that S1 P signaling via the EDG5 like receptor Miles Apart is essential for heart development. The presumed function of the EDG5 homologue in Zebrafish development and its expression in the heart suggests that it may play a critical role in the development and/or function of the cardiovascular system (Kupperman et al., (2000), Nature 406: 192-195).

WO 99/54351 discloses the use of EDG5 polypeptides and polynucleotides for diagnosis and therapy of diabetes and other diseases.

In order to analyze potential effects of EDG5 polymorphisms in humans, the V286A polymorphism (amino acid exchange Valine to Alanine at position 286 of the EDG5 protein) of the EDG5 protein in a clinical patient cohort enriched for cardiovascular outcomes was studied. So far, no data was available about the clinical effects of EDG5 variants in humans. (NCBI accession number for EDG5 protein sequence: NP_004221 and NCBI accession number for EDG5 nucleotide sequence: AF034780).

The present invention relates to a method of identifying an increase in risk for type II Diabetes mellitus and/or venous thrombosis/pulmonary embolism, wherein the presence of an amino acid exchange at position 286 from Valine (Val) to Alanine (Ala) in the EDG5 protein is determined in a probe.

The present invention further relates to a method of identifying an increase in risk for type II Diabetes mellitus and/or venous thrombosis/pulmonary embolism, wherein the presence of a variation in the nucleotide sequence of the EDG5 gene leading to an amino acid exchange at position 286 from Val to Ala in the EDG5 is determined in a probe.

The present invention further relates to a method of identifying an increase in risk for type II Diabetes mellitus and/or venous thrombosis/pulmonary embolism, wherein the presence of EDG-286-VA (EDG5 protein variants having Ala at position 286 of the protein as a consequence of nucleotide polymorphisms on one allele of the EDG5 gene) is determined in a probe.

Preferably, the probe is a cell or a body fluid, e. g. blood or an animal or human cell which was isolated from the human or animal body. The cell could also be a plant cell or a cell isolated from an animal.

Herein described is also a method of screening pharmaceuticals useful for treating and/or preventing type II Diabetes mellitus and/or venous thrombosis/pulmonary embolism, wherein a cell or cell extract is used that contains a EDG5 with
a) an amino acid exchange at position 286 from Val to Ala in the EDG5 protein,
b) a variation in the nucleotide sequence of the EDG5 gene leading to amino acid exchange at position 286 from Val to Ala in the EDG5 protein, or
c) EDG5-286-AA.

Furthermore, described is a method of adapting the dosage of a pharmaceutical useful for treating and/or preventing type II Diabetes mellitus and/or venous thrombosis/pulmonary embolism, wherein a human cell is tested for the presence of a EDG5 with
a) an amino acid exchange at position 286 from Val to Ala in the EDG5 protein,
b) a variation in the nucleotide sequence of the EDG5 gene leading to amino acid exchange at position 286 from Val to Ala in the EDG4 protein, or
c) EDG5-286-AA.

Also described herein is a method of selecting patients who will respond to type II Diabetes mellitus and/or venous thrombosis/pulmonary embolism pharmaceutical, wherein a probe of the respective patient is tested for the presence of EDG5 with
a) an amino acid exchange at position 286 from Val to Ala in the EDG5 protein,
b) a variation in the nucleotide sequence of the EDG5 gene leading to amino acid exchange at position 286 from Val to Ala in the EDG5 protein, or
c) EDG5-286-AA.

Described herein is a test kit for testing the presence of
a) an amino acid exchange at position 286 from Val to Ala in the EDG5 protein,
b) a variation in the nucleotide sequence of the EDG5 gene leading to amino acid exchange at position 286 from Val to Ala in the EDG5 protein, or
c) EDG5-286-AA.

The identification of the polymorphism in the nucleotide sequence of EDG5 leading to the amino acid exchange at position 286 from Val to Ala in the EDG5 protein can be used to predict increased or normal risk for type II Diabetes mellitus and/or venous thrombosis/pulmonary embolism. It can be used e. g. in
1) methods based on sequencing the nucleotide region of interest (e.g. pyrosequencing, sequencing methods using radio-labeled nucleotides, or nucleotides which are labeled with a fluorescent dye, analysis of sequence fragments with mass spectrometry);
2) methods based on the hybridization of nucleotide sequences to the region of interest (e.g. DNA microarrays);
3) methods based on analyzing amplification products of the nucleotide region of interest (e.g. TaqMan analysis).

The identification of the polymorphism in the protein sequence of EDG5 comprising the amino acid exchange at position 286 from Val to Ala in the EDG5 protein can be used to predict increased or normal risk for type II Diabetes mellitus and/or venous thrombosis/pulmonary embolism. It can be used, e.g. in
1) methods based on sequencing the protein region of interest (e.g. standard protein degradation, analysis of protein sequence fragments with mass spectrometry);
2) methods based on using anti-EDG5 antibodies against the region of interest (e.g. ELISA);
3) methods based on analyzing functional activity of EDG5 in in-vitro assays using e. g. human, animal, bacterial, or yeast cells.

The detection of genetic polymorphisms in the EDG5 gene, in particular EDG5-286-VA (EDG5 variants having Alanine at position 286 in the protein as a consequence of pholymorphisms at the corresponding position on one allele of the EDG5 gene), and the resulting protein by analyzing human DNA or EDG5 protein may be used (a) as genetic markers for preventive treatments to type II Diabetes mellitus and/or venous thrombosis/pulmonary embolism, (b) as a genetic marker for adaptation of drug dose, (c) as a genetic marker for drug screening set-up adaptation and (d) as a genetic marker for patient selection in phase/clinical studies.

The identification of the polymorphism in the nucleotide sequence of the EDG5 gene leading to the amino acid exchange at position 286 from Val to Ala in the EDG5 protein can be used to predict increased or normal risk type II Diabetes mellitus and/or venous thrombosis/pulmonary embolism. It can be used e. g. in
1) methods based on sequencing the nucleotide region of interest (e.g. pyrosequencing, sequencing methods using radio-labeled nucleotides, or nucleotides which are labeled with a fluorescent dye, analysis of sequence fragments with mass spectrometry);
2) methods based on the hybridization of nucleotide sequences to the region of interest (e.g. DNA microarrays);
3) methods based on analyzing amplification products of the nucleotide region of interest (e.g. TaqMan analysis).

The identification of the polymorphism in the protein sequence of EDG5 comprising the amino acid exchange at position 286 from Val to Ala can be used to predict increased or normal risk for type II Diabetes mellitus and/or venous thrombosis/pulmonary embolism. It can be used, e.g. in
1) methods based on sequencing the protein region of interest (e.g. standard protein degradation, analysis of protein sequence fragments with mass spectrometry);
2) methods based on using anti-EDG5 antibodies against the region of interest (e.g. ELISA);
3) methods based on analyzing functional activity of EDG5 in in-vitro assays using e. g. human, animal, bacterial, or yeast cells.

The detection of genetic polymorphisms in the EDG5 gene, in particular EDG5-286-VA (EDG5 variants having Alanine at position 286 of the protein as a consequence of pholymorphisms at the corresponding position on one allele of the EDG5 gene), and the resulting protein by analyzing human DNA and EDG5 protein may be used (a) as genetic markers for preventive treatments to prevent type II Diabetes mellitus and/or venous thrombosis/pulmonary embolism, (b) as a genetic marker for adaptation of drug dose, (c) as a genetic marker for drug screening set-up adaptation and (d) as a genetic marker for patient selection in phase/clinical studies.

### Examples

The known EDG5 polymorphism at the position 286 of the EDG5 protein (NCBI accession number for protein sequence: NP_004221 (Table 1A); NCBI accession number for nucleotide sequence: AF034780 (Table 1 B)) was analyzed in a patient cohort with or without cardiovascular events or endpoints.

### Example 1: Study subjects (study population):

The genomic DNA of 1140 patients was screened for SNPs in the EDG5 gene leading to the protein variants EDG5-V286A. The phenotype of this patient cohort has been described previously (Winkelmann et al. (2001) Pharmacogenomics, 2,1 - 73). Inclusion criteria have been: Caucasian individual of German ancestry, stable clinical condition (except for acute coronary syndrome [ACS]) and coronary angiogram. Exclusion criteria have been: acute illness other than ACS, chronic non-cardiac disease (i.e. rheumatic arthritis) and history of malignant disease within the previous five years. Basic characteristics of this patient cohort are outlined in Table 2.

### Example 2: SNP detection by sequencing and analysis

### Example 2.1:Amplification of genomic region with polymorphism of interest

### Amplification primers:

1. For the detection of nucleotide exchange Valine to Adenine at position 286 of the EDG5 gene sequence.
   Forward primer: 5'-TCCACTGTCCTGCCTCTCTAC -3' (SEQ ID NO. 1)
   Reverse primer: 5'-TCTCCATGAACCCCTCTGCC-3' (SQE ID NO. 2)

### PCR protocol for amplification:

All reagents are from Applied Biosystems (Foster City, USA): 20 ng of genomic DNA; 1 unit of TaqGold polymerase; 1 x Taq polymerase buffer; 500 µM of dNTP; 2,5 mM of MgCl2; 200 nM of each amplification primer pair (for sequence see Amplification primer pair 1. above); H₂O ad 5 µl.

### Amplification program for PCR/genotyping :

| | |
|---|---|
| 95°C x 10min | x 1 cycle |
| | |
| 95°C x 30sec | |
| 70°C x 30sec | x 2 cycles; |
| | |
| 95°C x 30sec | |
| 65°C x 30sec | x 2 cycles; |
| | |
| 95°C x 30sec | |
| 60°C x 30sec | x 2 cycles; |
| | |
| 95°C x 30sec | |
| 56°C x 30sec | |
| 72°C x 30sec | x 40 cycles; |
| | |
| 72°C x 10min | |
| 4°C x 30sec | x 1 cycle; |

### Example 2.2: Identification of polymorphism of interest

### Protocol for minisequencing and detection of polymorphisms:

All reagents are from Applied Biosystems (Foster City, USA). 2 µl of purified PCR product; 1,5 µl BigDye terminator kit; 200 nM of one sequencing primer (for sequence see forward or reverse Amplification primer 1. above); H2O ad 10 µl.

### Amplification program for sequencing :

| | |
|---|---|
| 96°C x 2' | x 1 cycle; |
| | |
| 96°C x 10" | |
| 55°C x 10" | |
| 65°C x 4' | x 30 cycles; |
| | |
| 72°C x 7' | |
| 4°C x 30" | x 1 cycle; |

### Analysis of sequencing products:

Sequences were analyzed first with sequencing analysis (Applied Biosystems, Foster City, USA) for raw data extraction, then processed with Phred (base caller), Phrap (assembler), polyphred (SNP caller) and Consed (results viewer). Phred, Phrap, Polyphred and Consed are software designed at WashU by Phil Green (http://www.genome.washington.edu).

### Example 2.3: Statistical approaches for genotype/phenotype correlation

All analyses were done with SAS statistical package (Version 6.12, SAS Institute GmbH, Heidelberg/Germany). For the detection of associations between genetic polymorphisms and a large number of clinical relevant parameters, descriptive statistics were computed (median, quartiles) and Wilcoxon-rank-sum-tests were performed. Wilcoxon-rank-sum-test is used for the comparison of two independent samples. The computation of the test statistic is based on ranks in the pooled sample. The search for associations between the SNPs and risk factors and diseases was done in a similar way. The Chi-Square-Test was performed and numbers and percentages were calculated to describe the data. The Chi-Square-Test is a statistical test for calculating the dependence of two variables. The values of the variables are contained in two or more classes. To analyze the association of those variables, a contingency table is used. This table contains as many rows as the number of realizations of the first variable and as many columns as the number of realizations of the second variable. Every cell contains a special patient's characteristic. To construct a test statistic, the differences of calculated and observed frequencies are computed.

After inspecting the results, relevant variables were selected. To take account of confounding co-variables, logistic regression was used to validate the results. The logistic regression method is used to analyze the influence of several explanatory variables on a certain response variable. The associated statistical test gives a p-value. The interpretation of this p-value is that there is a significant influence of the associated explanatory variable.

For a binary variable, the odds ratio has been calculated. The odds ratio is the ratio of the odds that an event will occur in one group to the odds that the event will occur in the other group.

### Example 2.4: Analyses

The following abbreviations are used:

EDG5-286-VV defines the group of individuals, in which both of the EDG5 alleles code for a EDG5 gene variant leading to Valine (V) at position 286 of the EDG5 protein, this group is homozygous for this EDG5 polymorphism at position 286 of the EDG5 protein. EDG5-286-VA defines the group of individuals, in which one of the EDG5 alleles codes for a EDG5 gene variant leading to Valine (V) at position 286 of the EDG5 protein and the other EDG5 allele codes for a EDG5 gene variant leading to Alanine (A) at position 286 of the EDG5 protein, this group is heterozygous for EDG5 polymorphism at position 286 of the EDG5 protein.

EDG5-286-AA defines the group of individuals, in which both of the EDG5 alleles code for a EDG5 gene variant leading to Alanine (A) at position 286 of the EDG5 protein, this group is homozygous for this EDG5 polymorphism at position 286 of the EDG5 protein.

The distribution of EDG5-286 variants in 1140 individuals is shown in Table 3. The EDG5-286-AA variant does not appear at all in this patient cohort. 2.1 % of all patients are carriers of the EDG5-286-VA variant and 97.8% of the patients are carriers of the EDG5-286-VV variant.

Patients carrying EDG5-286-VA show an increased association for type II diabetes mellitus (DM Type II) and venous thrombosis / pulmonary embolism (VT/PE) compared to EDG5-286-W patients. Statistical significance calculated with Chi-square test of the observed association is p-value = 0.001 for the association with DM Type II and p-value = 0.026 for the association with VT/PE (Tables 4A and 5A). Logistic regression models for analyzing the influence of confounding factors, such as myocardial infarction and hypertension resulted in a p-value = 0.0022 for the association of EDG5-286-VA with DM Type II to p-value and p-value = 0.0315 for the association of EDG5-286-VA with VT/PE (Tables 4B and 5B).

The odds ratios of decreased risk for DM Type II is 3.801 and for VT/PE 3.095 in individuals carrying the EDG5-286-VA variants compared to individuals with EDG5-286-VV variants (Tables 4C and 5C).

The EDG5-286-VA allele represents therefore a strong genetic marker to estimate decreased risk of DM Type II and venous thrombosis / pulmonary embolism.

### Table 1:

Protein sequence of EDG5 (ENDOTHELIAL DIFFERENTIATION GENE 5). The protein sequence accession number (NCBI protein database) of EDG5 is NP_004221 (A), the nucleotide sequence accession number (NCBI nucleotide database) is AF034780 (B) and the accession number for EDG5 information in OMIM (Online Mendelian Inheritance in Man^{™}) is 605111.

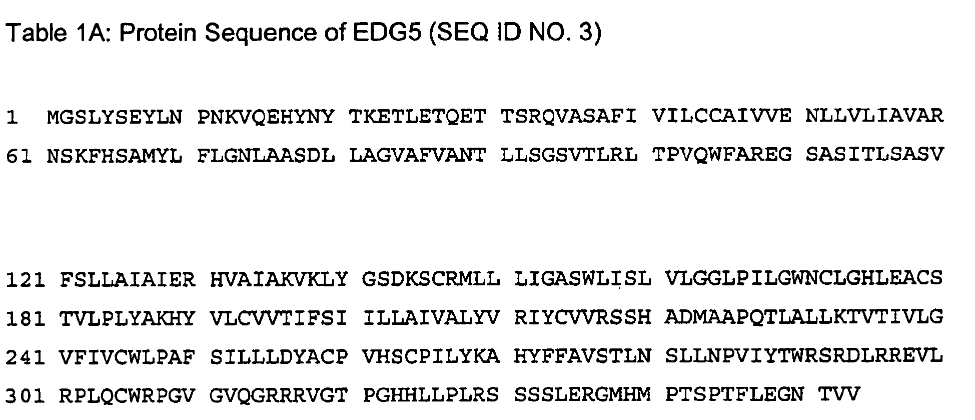

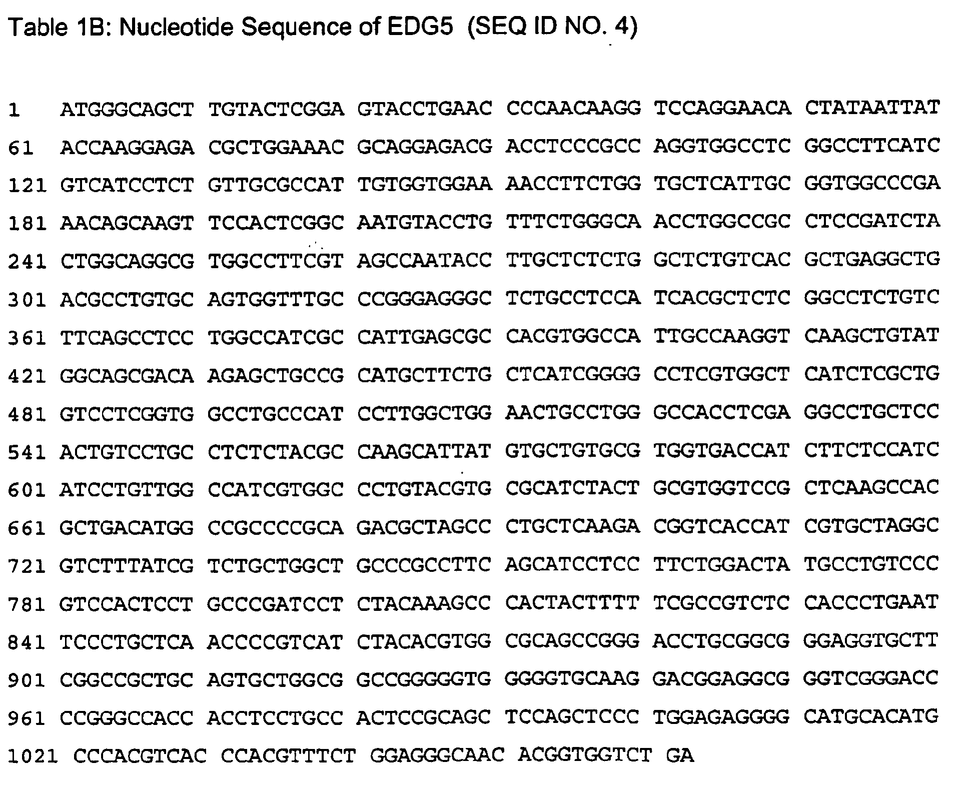

**Table 2: Basic characteristics of the patient cohort.**

| | | n | % |
|---|---|---|---|
| Total | | 1140 | |
| Gender | Female | 782 | 68.6 |
| | Male | 358 | 31.4 |
| Age* | | 63.3 | |
| | | (56.5-70.9) | |
| BMI* | | 27.9 | |
| (Body Mass Index) | | (25.0-30.3) | |
| Hypertension | | 670 | 58.8 |
| Smoker | | 731 | 64.1 |
| Diabetes | no oGT | 279 | 24.5 |
| | normal GT | 264 | 23.2 |
| | impaired GT | 236 | 20.7 |
| | diabetes new | 169 | 14.8 |
| | known diabetes | 192 | 16.8 |
| Diabetes | diabetes (total) | 361 | 31.7 |
| | no diabetes (total) | 779 | 68.3 |
| Stable CAD | CCS 1 | 457 | 40.1 |
| | CCS 2 | 409 | 35.9 |
| | CCS 3 | 191 | 16.8 |
| | CCS 4 | 83 | 7.3 |
| Unstable CAD | no ACS (noCAD/stable CAD/MI>15d) | 743 | 65.2 |
| | tropT-UA (no acute MI) | 250 | 21.9 |
| | tropT+UA (no clinical MI) | 42 | 3.7 |
| | post acute MI (1-15 d) | 105 | 9.2 |

| | | | |
|---|---|---|---|
| *Median and Quartiles (Q1-Q3) | | | |

**Table 3:**

| Distribution of EDG5 variants in the analyzed patient cohort. | |
|---|---|
| | Number of patients (%) |
| **EDG5-286-VV** | 1116 (97.9%) |
| **EDG5-286-VA** | 24 (2.1%) |
| **EDG5-286-AA** | 0 (0%) |

**Table 4A:**

| Association of EDG5-286 variants with type II diabetes mellitus calculated by Chi-square test. An increased frequency of EDG5-286-VA carriers in the type II diabetes mellitus positive group could be observed compared to EDG5-286-W patients. | | | |
|---|---|---|---|
| | **Type II Diabetes Mellitus** | | |
| | Number of patients without T-II-DM (%) | Number of patients with T-II-DM (%) | p-value |
| **EDG5-286-VA** | 9 (37.5%) | 15 (62.5%) | **0.001** |
| **EDG5-286-VV** | 770 (69.0%) | 346 (31.0%) | |

**Table 4B:**

| Calculation of statistical significance for EDG5-286-VA association with type II diabetes mellitus by logistic regression. | |
|---|---|
| | **p-value (logistic regression)** |
| **EDG5-286-VA** | **0.0022** |
| Male gender | 0.9926 |
| Smoker | 0.7159 |
| Arterial hypertension | 0.0001 |
| MI | 0.0044 |
| ACS | 0.5657 |
| Tot. Cholesterol >=240 or drug history | 0.4831 |
| Venous thrombosis / pulmonary embolism | 0.5843 |

**Table 4C:**

| Calculation of odds ratios for the risk of having type II diabetes mellitus of EDG5-286-VA patients compared to EDG5-286-VV patients. | | | | |
|---|---|---|---|---|
| | | 95%-confidence interval | | |
| | odds ratio | lower | Upper | p-value |
| **Type II Diabetes Mellitus** | 3.801 | 1.614 | 8.949 | **0.0022** |

**Table 5A:**

| Association of EDG5-286 variants with venous thrombosis / pulmonary embolism calculated by Chi-square test. An increased frequency of EDG5-286-VA carriers in the venous thrombosis / pulmonary embolism positive group could be observed compared to EDG5-286-VV patients. | | | |
|---|---|---|---|
| | **Venous thrombosis / pulmonary embolism (VT/PE)** | | |
| | Number of patients without VT/PE (%) | Number of patients with VT/PE (%) | p-value |
| **EDG5-286-VA** | 19 (79.2%) | 5 (20.8%) | **0.026** |
| **EDG5-286-VV** | 1030 (93.0%) | 78 (7.0%) | |

**Table 5B:**

| Calculation of statistical significance for EDG5-286-VA association venous thrombosis / pulmonary embolism (VT/PE) by logistic regression. | |
|---|---|
| | **p-value (logistic regression)** |
| **EDG5-286-VA** | **0.0351** |
| Male gender | 0.0004 |
| Smoker | 0.0965 |
| Arterial hypertension | 0.7338 |
| Ml | 0.8597 |
| ACS | 0.9270 |
| Diabetes mellitus type II | 0.5796 |
| Tot. Cholesterol >=240 or drug history | 0.9452 |

**Table 5C:**

| Calculation of odds ratios for the risk of having venous thrombosis / pulmonary embolism of EDG5-286-VA patients compared to EDG5-286-VV patients. | | | | |
|---|---|---|---|---|
| | | 95%-confidence interval | | |
| | odds ratio | lower | Upper | p-value |
| **Venous thrombosis / Pulmonary embolism** | 3.095 | 1.082 | 8.853 | **0.0351** |

## Claims

1. A method of identifying an increase in risk for type II Diabetes mellitus and/or venous thrombosis/pulmonary embolism, wherein the presence of amino acid exchange at position 286 from Val to Ala in the EDG5 protein is determined in a probe.

2. A method of identifying an increase in risk for type II Diabetes mellitus and/or venous thrombosis/pulmonary embolism, wherein the presence of a variation in the nucleotide sequence of the EDG5 gene leading to amino acid exchange at position 286 from Val to Ala in the EDG5 protein is determined in a probe.

3. A method of identifying an increase in risk for type II Diabetes mellitus and/or venous thrombosis/pulmonary embolism, wherein the presence of EDG5-286-VA (EDG5 variants having Ala at position 286 of the protein as a consequence of nucleotide polymorphisms on one allele of the EDG5 gene) is determined in a probe.

4. A method according to one of the forgoing claims, wherein the probe is a cell.

5. A method according claim 4, wherein the cell is a human cell isolated from the human body.

## Patentansprüche

1. Verfahren zum Identifizieren eines erhöhten Risikos für Diabetes mellitus Typ II und/oder Venenthrombose/Lungenembolie, wobei das Vorliegen eines Aminosäureaustausches in Position 286 von Val gegen Ala im EDG5-Protein in einer Probe bestimmt wird.

2. Verfahren zum Identifizieren eines erhöhten Risikos für Diabetes mellitus Typ II und/oder Venenthrombose/Lungenembolie, wobei das Vorliegen einer Variation in der Nukleotidsequenz des EDG5-Gens, die zu einem Aminosäureaustausch in Position 286 von Val gegen Ala im EDG5-Protein führt, in einer Probe bestimmt wird.

3. Verfahren zum Identifizieren eines erhöhten Risikos für Diabetes mellitus Typ II und/oder Venenthrombose/Lungenembolie, wobei das Vorliegen von EDG5-286-VA (EDG5-Varianten mit Ala in Position 286 des Proteins aufgrund von Nukleotidpolymorphismen an einem Allel des EDG5-Gens) in einer Probe bestimmt wird.

4. Verfahren nach einem der vorhergehenden Ansprüche, wobei es sich bei der Probe um eine Zelle handelt.

5. Verfahren nach Anspruch 4, wobei es sich bei der Zelle um eine aus dem menschlichen Körper isolierte menschliche Zelle handelt.

## Revendications

1. Procédé d'identification d'une hausse du risque de développer un diabète sucré de type II et/ou une thrombose veineuse/embolie pulmonaire, selon lequel la présence d'un échange d'acides aminés en position 286 de val en Ala dans la protéine EDG5 est déterminée dans un échantillon.

2. Procédé d'identification d'une hausse du risque de développer un diabète sucré de type II et/ou une thrombose veineuse/embolie pulmonaire, selon lequel la présence d'une variation dans la séquence nucléotidique du gène d'EDG5 conduisant à un échange d'acides aminés en position 286 de Val en Ala dans la protéine EDG5 est déterminée dans un échantillon.

3. Procédé d'identification d'une hausse du risque de développer un diabète sucré de type II et/ou une thrombose veineuse/embolie pulmonaire, selon lequel la présence de EDG5-286-VA (variants d'EDG5 possédant une Ala en position 286 de la protéine en conséquence de polymorphismes nucléotidiques sur un allèle du gène d'EDG5) est déterminée dans un échantillon.

4. Procédé selon l'une des revendications précédentes, selon lequel l'échantillon est une cellule.

5. Procédé selon la revendication 4, selon lequel la cellule est une cellule humaine isolée à partir du corps humain.
